# EUROPEAN PATENT APPLICATION

(11) **EP 1 674 577 A1**
(43) Date of publication of application: **28.06.2006**
(21) Application number: 04107022.8
(22) Date of filing: 23.12.2004
(51) Int. Cl.: C12N 15/80

(54) **Method for expressing a nucleotide sequence in fungi**

(71) Applicant: BASF Aktiengesellschaft, 67056 Ludwigshafen (DE)
(72) Inventor: Rether, Jan, Dr., 67655 Kaiserslautern (DE); Lscour, Thierry, Dr., 76297 Stutensee (DE); Freund, Annette, Dr., 67117 Limburgerhof (DE); Jabs, Thorsten, Dr., 67454 Hassloch (DE); Schiffer, Helmut, Dr., 67483 Grossfischlingen (DE)
(74) Representative: Fitzner, Uwe

(57) **Abstract**

The invention relates to a method for expressing a nucleotide sequence in fungi, novel promoters, gene constructs, expression cassettes and vectors comprising said promoters. The invention furthermore relates to the use of said promoters in a process for the production of fine chemicals and in a process for identifying targets for fungicides.

## Description

The invention relates to a method for expressing a nucleotide sequence in fungi, novel promoters, gene constructs, expression cassettes and vectors comprising said promoters. The invention furthermore r elates to the use of said promoters in a process for the production of fine chemicals and in a process for identifying targets for fungicides.

The ability to control the level and timing of the expression of specific genes in fungi has many useful applica tions. Development of these applications is based on the isolation of well characterised promoters which will drive the expression of fused genes in a predictable manner. Regulated promoters from a number of different yeast species have already found com mercial application, these include the thiamine repressible nmt1 promoter from the fission yeast Schizosaccharomyces pombe (Invitrogen SpECTRA ® Expression System; Maundrell, 1993; Basi et al., 1993), the methanol inducible AOX1 (alcohol oxidase) promoter from Pichia pastoris (Ellis et al., 1985; Koutz et al., 1989; Tschopp et al., 1987) the glucose -repressed and galactose -induced GAL1 and GAL10 promoters (West et al., 1984; Yocum et al., 1984). Other yeast promoters that have been employed in protein puri fication include the glucose -repressed alcohol dehydrogenase ADH2 promoter (Price et al., 1990), the SOR1 sorbitol induced promoter (McGonigal et al., 1998) the copper sulfate induced metallothionein CUP1 promoter (Macreadie, 1989), the sucrose induced INV1 promoter (lacovoni et al., 1999), and the phosphate limitation induced PHO5 promoter (Choo et al., 1985). Among the filamentous fungi, the isolation of strong inducible promoters from xylanase and cellulase encoding genes in Trichoderma reesei the fungus, together with a high capacity for secretion, have made this an attractive host for heterologous protein production (reviewed by Mach and Zeilinger, 2003). Aspergillus species have also been considered useful hosts for protein production using inducible p romoters related to those described above, for example the alcA promoter from Aspergillus nidulans (Waring et al., 1989) as well as the starch induced glaA promoter from Aspergillus niger (Carrez et al., 1990). Outside of the field of protein production th e tightly controlled MET3 (methionine repressible) promoter of Candida albicans has been employed in the analysis of essential genes by controlled switching between viable and non -viable states (Care et al., 1999; Mao et al., 2002). Regulated promoters have recently also found use in the controlled silencing of target genes, for example using the xylP promoter in Penicillium chrysogenum (Zadra et al., 2000).

Promoters for the expression of lipase in Fusarium venenatum are disclosed in US 6,518,044.

Although several promoters have proven to be useful for expression of genes, there still is a need for promoters for controlling the expression of heterologous and homologous genes. Specially there is a need for new, strongly inducible promoters in fungi, pref erably filamentous fungi.

It is an object of the present invention to provide a method for expressing a nucleotide sequence in fungi wherein the expression is driven by tightly regulated promoters, especially promoters that can be induced independently of the promoters already available.
It is a further object of the invention to provide tightly regulated promoters as tools for heterologous or homologous fine chemicals production or for gene function analysis in a broader range of fungi.

The objects of the invention are achieved by a method for expressing a nucleotide sequence in fungi / a fungal cell, said method comprising transforming a fungi with a transformation gene construct comprising
I) at least one nucleotide sequence and
II) a promoter operably linked to the nucleotide sequence I), comprising
   a) a nucleotide sequence as set forth in SEQ ID NO:1 and/or
   b) a nucleotide sequence as set forth in SEQ ID NO:8 and/or
   c) a fragment of (a) or (b) that retains the promoter activity of SEQ ID NO:1 and/or SEQ ID NO:8 and/or
   d) functional equivalent nucleic acid molecule of the nucleic acid sequence set forth in (a) to (c) which has at least 50% identity with the nucleic acid molecule of (a) to (c) and/or
   e) nucleic acid molecule which encompasses a nu cleic acid molecule which is obtained by amplifying nucleic acid molecules from a cDNA library or a genomic library using the primers in SEQ ID NO: 2 and 3 or SEQ ID NO: 4 and 5 or SEQ ID NO: 9 and 10 and/or
   f) nucleic acid molecule which is obtainable by screening a suitable nucleic acid library under stringent hybridization conditions with a probe comprising one of the sequences of the nucleic acid molecule of (a) to (e) or with a fragment thereof having at least 10 -15 nt, preferably 16-20 nt, 21-30 nt, 31-50 nt, 51-100 nt, 101-200 nt or 201-500 nt of the nucleic acid molecule characterized in (a) to (e)
and
wherein the promoter II) drives the expression of the nucleotide sequence I) in a fungi or fungal cell.

The term "transformation" means a process for introducing heterologous (or homologous) DNA into a cell or fungus. Transformed cells or fungi are understood to encompass not only the end product of a transformation process, but also transgenic progeny thereof.

In the case of fungi, the skilled wor ker will find suitable methods in the tex tbooks by Sambrook, J. et al. (1989) "Molecular cloning: A laboratory manual", Cold Spring Harbor Laboratory Press, by F.M. Ausubel et al. (1994) "Current protocols in molecular biology", John Wiley and Sons, by D.M Glover et al., DNA Cloning Vol.1, (1995), IRL Press (ISBN 019 -963476-9), by Kaiser et al. (1994) Methods in Yeast G enetics, Cold Spring Habor Laboratory Press or Guthrie et al. "Guide to Yeast Genetics and Molecular Biology", Methods in Enzymology, 1994,Academic Press. In the case of the transformation of filamentous fungi, methods of choice are firstly the generation of protoplasts and transformation with the aid of PEG (Wiebe et al. (1997) Mycol. Res. 101 (7): 971-877; Proctor et al. (1997) Microbiol. 143, 2538-2591) and secondly the transformation with the aid of Agrobacterium tumefaciens (de Groot et al. (1998) Nat. Biotech. 16, 839-842).

The term "transgenic" means stably transformed with a recombinant DNA molecule that preferably comprises a suitab le promoter operatively / functionaly linked to a DNA sequence of interest.

"Promotor" refers to a DNA sequence in a gene, usually upstream (5') to its coding sequence, which controls the expression of the coding sequence by providing the recognition for R NA polymerase and other factors required for proper transcription. The promoter is a DNA sequence that binds RNA polymerase and directs the polymerase to the correct downstream transcriptional start site of a nucleic acid sequence encoding a polypeptide to initiate transcription. RNA polymerase effectively catalyzes the assembly of messenger RNA complementary to the appropriate DNA strand of the coding region. The term "promoter" will also be understood to include the 5' non -coding region (between promoter and translation start) for translation after transcription into mRNA, cis -acting transcription control elements such as enhancers, and other nucleotide sequences capable of interacting with transcription factors.
A promoter may also contain DNA sequences that are involved in the binding of protein factors which control the effectiveness of transcription initiation in response to physiological or developmental conditions. One can distinguish "constitutive promoters", which refers to those promoters that dir ect gene expression in all tissues and at all times and "inducible promoters", which refer to those promoters which turn on the description of a gene in the presence of an inducer.

In a prefered embodiment the promoters of the present invention are induci ble promoters. The term "inducible" as applied to a promoter is well understood by those skilled in the art. In essence, expression under the control of an inducible promoter is "switched on" or increased in response to an applied stimulus or inducer (whic h may be generated within a cell or provided exogenously). The nature of the stimulus varies between promoters.
Some inducible promoters cause little or undetectable levels of expression (or no expression) in the absence of the appropriate stimulus/ induce r. Other inducible promoters cause detectable constitutive expression in the absence of the stimulus. Whatever the level of expression is in the absence of the stimulus, expression from any inducible promoter is increased in the presence of the correct sti mulus. The preferable situation is where the level of expression increases upon application of the relevant stimulus by an amount effective to alter a phenotypic characteristic. Thus an inducible (or "switchable") promoter may be used which causes a basic level of expression in the absence of the stimulus which level is too low to bring about a desired phenotype (and may in fact be zero). Upon application of the stimulus, expression is increased (or switched on) to a level which brings about the desired phe notype.

The method of the present invention include further promoters which are hybrid promoters of fragments of two or more promoters, in which one or more fragments are fragments of nucleotide acid molecules as set forth in SEQ ID NO: 1 and SEQ ID NO: 8, wherein each fragment contributes to the activity of the hybrid promoter. The other fragments of the hybrid promoters are preferably from different promoter.

The method of the present invention include further promoters which are tandem promoter comprisin g one or more copies of one or more nucleic acid sequences selected from the group comprising
a) a nucleotide sequence as set forth in SEQ ID NO:1 and/or
b) a nucleotide sequence as set forth in SEQ ID NO:8 and/or
c) a fragment of (a) or (b) that retains the promoter activity of SEQ ID NO:1 and/or SEQ ID NO:8 and/or
d) functional equivalent nucleic acid molecule of the nucleic acid sequence set forth in (a) to (c) which has at least 50% identity with the nucleic acid molecule of (a) to (c) and/or
e) nucleic acid molecule which encompasses a nucleic acid molecule which is obtained by amplifying nucleic acid molecules from a cDNA library or a genomic library using the primers in SEQ ID NO: 2 and 3 or SEQ ID NO: 4 and 5 or SEQ ID NO: 9 and 10 and/or
f) nucleic acid molecule which is obtainable by screening a suitable nucleic acid library under stringent hybridization conditions with a probe comprising one of the sequences of the nucleic acid molecule of (a) to (e) or with a fragment thereof having at least 10-15 nt, preferably 16 -20 nt, 21-30 nt, 31-50 nt, 51-100 nt, 101-200 nt or 201-500 nt of the nucleic acid molecule characterized in (a) to (e).

For the purposes of the invention, as a rule the plural is intended to encompass the singular, the term "fungi " is intended to encompass "a fungal cell" and vice versa.

The term "hybrid promoter" is defined herein as parts of two more promoters that are fused together to generate a sequence that is a fusion of the two or more promoters, which is operably linked to a coding sequence and mediates the transcription of the coding sequence into mRNA.

The term "tandem promoter" is defined herein as two or more promoter sequences each of which is operably linked to a coding sequence and mediates the transcription of th e coding sequence into mRNA.

The term "operably linked" refers to nucleic acid sequences on a single nucleic acid molecule which are associated so that the function of one is affected by the other. For example, the above mentioned promoter is operably link ed with a structural gene when it is capable of affecting the expression of that structural gene (i.e., that the structural gene is under the transcriptional control of the promoter).
A operable or functional linkage is understood to mean the sequential a rrangement of promoter and coding sequence, of coding sequence and terminator or of promoter, coding sequence and terminator in such a manner that each of the regulatory elements can, upon expression of the coding sequence, fulfil its function for the reco mbinant expression of the nucleic acid sequence. Direct linkage in the chemical sense is not necessarily required for this purpose. Preferred arrangements are those in which the gene of interest to be expressed recombinantly is positioned downstream of t he sequence which acts as promoter, so that the two sequences are linked covalently to each other. The distance between the promoter sequence and the nucleic acid sequence to be expressed recombinantly is preferably less than 100 base pairs, especially preferably less than 50 base pairs, and very especially preferably less than 10 base pairs. The distance between the terminator sequence and the nucleic acid sequence to be expressed recombinantly is preferably less than 100 base pairs, especially preferably less than 50 base pairs, and very especially preferably less than 10 base pairs. However, further sequences which, for example, exert the function of a linker with certain restriction enzyme cleavage sites, or of a signal peptide, may also be positioned be tween the two sequences.

The terms "fragment", "fragment of a sequence" or "part of a sequence" mean a truncated sequence of the original sequence r eferred to. The truncated nucleic acid sequence can vary widely in length; the minimum size being a s equence of sufficient size to provide a sequence or sequence fragment with at least 15, 20, 21, 22, 23, 24, 25, 26, 27, 28, 29, 30 bp in length with at least 90, 91, 92, 93, 94, 95, 96, 97, 98 or 99 % identity preferably 100 % identity with a fragment of the nucl eic acid molecules of the invention for example with the sequences shown in SEQ ID NO: 1 or SEQ ID NO: 8. Said truncated sequences can as mentioned vary widely in length from 15 bp up to 2 kb or more, advantageously the sequences have a minimal length of 15, 20, 25, 30, 35 or 40 bp, while the maximum size is not crit ical. 100, 200, 300, 400, 500 or more base pair fragments can be used. In some applications, the maximum size us ually is not substantially greater than that required to provide the complete fun ction(s) of the nucleic acid sequences of the invention.

In an prefered embodiment the fungi / fungal cell is a filamentous fungi / fungal cell, preferably an Acremonium, Aspergillus, Aureobasidium, Cryptococcus, Filibasidium, Fusarium, Humicola, Magnapor the, Mucor, Myceliophthora, Neocallimastix, Neurospora, Paecilomyces, Penicillium, Schizophyllum, Talaroinyces, Thermoascus, Thielavia, Tolypocladium, or Trichoderma and/or Alternaria kikuchiana, Alternaria mali, Alternaria solani, Ashbya gossypii, otryti s cinerea, Cercospora beticola, Cercospora fuligena, Cercospora kaki, Cladosporum carpophilum, Cochliobolus heterostrophus, Colletotrichum fragariae, Colletotrichum heterostrophus, Colletotrichum lagenarium, Corynespora melonis, Diaporthe citri, Diplocar pon rosae, Elsinoe fawcetti, Erisyphe graminis, Leveillula taurica, Fusarium culmorum Fusarium nivale, Fusarium graminearum, Gloedes pomigena, Gloesporium kaki, Glomerella cingulata, Gymnosporangium yamadae, Leptothyrium pomi, Magnaporthe grise, Mycospha erella pomi, Mycosphaerella nawae, Neurospora crassa, Peronospora destructor, Peronospora spinaciae, Phaeoisariopsis vitis, Phyllactinia kakicola, Physalospora canker, Phytophthora citrophithora, Phytophthora investans, Phytophthora porri, Plasmopora viti cola, Podosphaera leucotricha, Podosphaera tridactyla, Pomophis sp., Pseudocercorsporella herpotrichoides, Pseudoperonospora cubensis, Puccinia allii, Puccinia recondita, Puccinia horiana, Pyricularia oryzae, Uncinula necator, Sclerotinia cinerea, Sclerot inia mali, Sclerotinia sclerotiorum, Septoria tritici, Sphaerotheca fuliginea, Sphaerotheca humuli, Sphaerotheca pannosa, Spaceloma ampelina, Stagnospora nodorum, Typhula ishikariensis, Typhula incarnata, Ustilago maydis, Venturia inaequalis, Venturia nashicola. Weitere bevorzugte Pilzstämme sind Aspergillus, Trichoderma, Neurospora, Fusarium, Beauveria, Pyrenophora teres, Saccharomyces (for example Saccharomyces cerevisiae), Pichia (like Pichia pastoris, Pichia methanolica), Magnaporthe, Pyrialeria or furhter fungi as disclosed in Indian Chem Engr. Section B. Vol 37, No 1,2 (1995).

In an prefered embodiment the fungi is a Magnaporthe species, wherein Magnaporthe species include Magnaporthe rhizophila, Magnaporthe salvinii, Magnaporthe poae and Magnaporthe grisea, preferably Magnaporthe grisea.

In another prefered embodiment the promoter as set forth in SEQ ID NO:1 and SEQ ID NO: 4 are from Magnaporthe grisea.

The present invention further relates to a promoter from Magnaporthe grisea comprising
a) a nucleotide sequence as set forth in SEQ ID NO:1 and/or
b) a nucleotide sequence as set forth in SEQ ID NO:8 and/or
c) a fragment of (a) or (b) that retains the promoter activity of SEQ ID NO:1 and/or SEQ ID NO:8 and/or
d) functional equivalent nucleic acid molecule of the nucleic acid sequence set forth in (a) to (c) which has at least 50% identity with the nucleic acid molecule of (a) to (c) and/or
e) nucleic acid molecule which encompasses a nucleic acid molecule which is obtained by amplifying nuclei c acid molecules from a cDNA library or a genomic library using the primers in SEQ ID NO: 2 and 3 or SEQ ID NO: 4 and 5 or SEQ ID NO: 9 and 10 and/or
f) nucleic acid molecule which is obtainable by screening a suitable nucleic acid library under stringent hybridization conditions with a probe comprising one of the sequences of the nucleic acid molecule of (a) to (e) or with a fragment thereof having at least 10 -15 nt, preferably 16 -20 nt, 21-30 nt, 31-50 nt, 51-100 nt, 101-200 nt or 201-500 nt of the nuclei c acid molecule characterized in (a) to (e).

In one embodiment the invention relates to a gene construct comprising
I) at least one nucleotide sequence and
II) a promoter as disclosed above functionally linked to the nucleotide sequence I),
wherein the promoter II) drives the expression of the nucleotide sequence I) in a fungi /fungal cell.

In another embodiment the invention relates to an expression cassette comprising
I) at least one nucleotide sequence and
II) a promoter as disclosed above,
III) at least one functional element
wherein all three elements are functionally linked together.

The term "expression cassette" as used herein means a DNA sequence capable of directing expression of a particular nucleotide sequence in an appropriate host cell , comprising a promoter operatively linked to the nucleotide sequence of interest which is operatively linked to termination signals. It also typically comprises sequences required for proper translation of the nucleotide sequence. The coding region usuall y codes for a protein of interest but may also code for a functional RNA of interest, for example antisense RNA or a nontranslated RNA, in the sense or antisense direction. The expression cassette comprising the nucleotide sequence of interest may be chime ric, meaning that at least one of its components is heterologous with respect to at least one of its other components. The expression cassette may also be one which is naturally occurring but has been obtained in a recombinant form useful for heterologous expression. Typically, however, the expression cassette is heterologous with respect to the host, i.e., the particular DNA sequence of the expression cassette does not occur naturally in the host cell and must have been introduced into the host cell or an ancestor of the host cell by a transformation event. The expression of the nucleotide sequence in the expression cassette may be under the control of a constitutive promoter or of an inducible promoter which initiates transcription only when the host cell is exposed to some particular external stimulus. In the case of a multicellular organism, such as an insect, the promoter can also be specific to a particular tissue or organ or stage of development.

Besides promoter the expression cassettes may also conta in functional el ements. "Functional elements" are herein understood as meaning by way of example but not by limitation origins of replication, selection markers and reporter genes, functionally linked to the nucleic acid sequence in accordance with the inv ention direct or by means of a linker optionally comprising a protease cleavage site.

"Selection markers" confer resistance to antibiotics or other toxic compounds: examples which may be mentioned in this co ntext are the neomycin phosphotransferase gene, which confers resistance to the aminoglycoside antibiotics neomycin (G 418), kan amycin, paromycin (Deshayes A et al., EMBO J. 4 (1985) 2731 -2737), the sul gene encoding a mutated dihydropteroate synthase (Gue rineau F et al., Plant Mol Biol. 1990; 15(1):127 - 136), the hygromycin B phosphotransferase gene (Gen Bank Accession NO: K 01193) and the she ble resistance gene, which confers resistance to the bleom ycin antibiotics, e.g. zeocin. Further examples of selection marker genes are genes which confer resistance to 2-deoxyglucose-6-phosphate (WO 98/45456) or phosphinothricin and the like, or those which confer a resistance to ant imetabolites, for example the dhfr gene (Reiss, Plant Physiol. (Life Sci. Adv.) 13 (1994) 142 -149). Examples of other genes which are suitable are trpB or hisD (Hartman SC and Mulligan RC, Proc Natl Acad Sci U S A. 85 (1988) 8047-8051). Another suitable gene are the mannose phosphate isomerase gene (WO 94/20627) or the ODC (ornithine decarboxylase) gene (McConlogue, 1987 in: Current Comm unications in Molecular Biology, Cold Spring Harbor Laboratory, Ed.) or the Aspergillus terreus deaminase (Tamura K et al., Bi osci Biotechnol Biochem. 59 (1995) 2336-2338) . Further selection markers are the Ura3 gene, the IIv2 gene, P -lactamase gene, the neomycin phosphotransferase gene, the hygromycin phosphotransferase gene, or the BASTA (= gluphosinate) resistance gene

Ori or "origin of replication" ensure the multiplication of the expression cassettes or vectors according to the invention in yeast, for example the ARS1 ori in yeast (Nucleic Acids Research, 2000, 28(10): 2060 -2068).

By a "reporter gene" is meant a gene whose expression may be assayed. Reporter genes encode readily quantifiable proteins. Using these genes, an assessment of transformation efficacy or of the site or time of expression can be made via growth, fluorescence, chemoluminescence, bioluminescence or resistance assay or via photometric measurement (intrinsic color) or enzyme activity. Very especially preferred in this context are reporter proteins (Schenborn E, Groskreutz D. Mol. Biotechnol. 1999; 13 (1): 29-44) such as the "green fluorescence protein" (GFP) (Gerdes HH and Kaether C, FEBS Lett. 1996; 389 (1) : 44 -47; Chui WL et al. , Curr. Biol. 1996,6 : 325-330; Leffel SM et al., Biotechniques. 23 (5): 912 -8,1997), chloramphenicol acetyl transferase, a luciferase (Giacomin, Plant Sci. 1996,116 : 59 -72; Scikantha, J. Bact. 1996,178 : 121; Millar et al., Plant Mol. Biol. Rep. 1992 10: 324 -414), and luciferase genes, in gen eral-galactosidase or -glucuronidase (Jefferson et al. , EMBO J. 1987,6, 3901 -3907). Further reporter genes include, without limitation, genes encoding beta -galactosidase, beta - glucoronidase, beta -glucosidase, and invertase, amino acid biosynthetic genes, e.g., the yeast LEU2, HIS3, LYS2, TRP1 genes, nucleic acid biosynthetic genes, e.g., the yeast ADE2 genes, or any surface antigen gene for which specific antibodies are available. Reporter genes may encode also any protein that provides a phenotypic mark er, for example, a protein that is necessary for cell growth or viability, or a toxic protein leading to cell death, or the reporter gene may encode a protein detectable by a color assay leading to the presence or absence of color. Alternatively, a reporte r gene may encode a suppressor tRNA, the expression of which produces a phenotype that can be assayed. A reporter gene according to the invention includes elements (e.g., all promoter elements) necessary for reporter gene function.

All embodiments of the a bove-referred expression cassettes will herein after be referred to as "expression cassette according to the invention".

The expression cassettes according to the invention are also understood as meaning analogs which can be brought about, for example by a combination of the individual nucleic acid sequences on a polynucleotide (multiple constructs), on a plurality of polynucleotides in a cell (cotransformation) or by sequential transformation.

In a further embodiment the expression cassette additionally co mprises
IV) a terminator which facilitates transcription termination, preferably in fungi, which is functionally linked to the nucleic acid sequence III).

The terminator facilitates transcription termination preferably in filamentous fungi. Examples of su itable terminators that are functionally linked to the coding region of the expression cassette are the AOX1 -, nos-, PGK-, TrpC-or the CYCI-terminator (Degryse et al., Yeast 1995 Jun 15; 11 (7): 629 -40; Brunelli et al. Yeast 1993 Dec9 (12): 1309 -18; Frisch et al., Plant Mol. Biol. 27 (2), 405 -409 (1995); Scorer et al., Biotechnology (N. Y.) 12 (2), 181-184 (1994), Genbank acc. number Z46232; Punt et al. , (1987) Gene 56 (1), 117-124) ), preferably the CYC1 -or nos-terminator, more preferably the nos -terminator.

The nucleic acid sequence I) can be also functionally linked to an affinity tag to purify the encoded protein.

The term "affinity tag" denotes a peptide or polypeptide whose coding nucleic acid sequence can be fused to the nucleic acid sequenc e I) either directly or using a linker, by classical cloning techniques. The affinity tag serves to isolate the recombinant target protein by means of affinity chromatography. The abovementioned linker can optionally comprise a protease cleavage site (for example for thrombin or factor Xa), whereby the affinity tag can be cleaved off from the target protein, as required. Examples of customary affinity tags are the"his-tag", for example from Quiagen, Hilden,"strep -tag","myc-tag" (Invitrogen, Carlsberg), New England Biolab's tag which consists of a chitin binding do - main and an intein, and what is known as the CBD -tag from Nova gen.

In one embodiment the invention relates to a transformation vector comprising
I) at least one nucleotide sequence and
II) a promoter as disclosed above functionally linked to the nucleotide sequence I),
wherein the promoter II) drives the expression of the nucleotide sequence I) in a fungi /fungal cell.

In a preferd embodiment the transformation vector comprises an expressio n cassette according as disclosed above.

Preferably, the vector also comprises a multiple cloning site comprising an appropriate restriction enzyme site. Appropriate restriction sites are well known by the skilled artisan. In a further preferred embodim ent, the plasmid vector additionally comprises a TA -cloning site to facilitate the overall cloning procedure.

The functional equivalents of the nucleic acid sequence set forth in SEQ ID NO: 1 are nucleic acid molecules with a sequence that has at least an identity of 50%, 51 %, 52%, 53%, 54%, 55%, 56%, 57%, 58%, 59%, 60%, 61%, 62%, 63%, 64% or 65% or preferably of 66%, 67%, 68%, 69%, 70%, 71%, 72%, 73%, 74%, 75%, 76%, 77%, 78% or 79% more preferably of 80%, 81 %, 82%, 83%, 84%, 85%, 86%, 87%, 88%, 89% or 9 0% most preferably of 91%, 92%, 93%, 94%, 95%, 96%, 97%, 98% or 99% with SEQ ID NO : 1.

The functional equivalents of the nucleic acid sequence set forth in SEQ ID NO: 8 are nucleic acid molecules with a sequence that has at least an identity of 50%, 51 %, 52%, 53%, 54%, 55%, 56%, 57%, 58%, 59%, 60%, 61%, 62%, 63%, 64% or 65% or preferably of 66%, 67%, 68%, 69%, 70%, 71%, 72%, 73%, 74%, 75%, 76%, 77%, 78% or 79% more preferably of 80%, 81%, 82%, 83%, 84%, 85%, 86%, 87%, 88%, 89% or 90% most preferably of 91% , 92%, 93%, 94%, 95%, 96%, 97%, 98% or 99% with SEQ ID NO : 8.

"Functional equivalents" in the present context describe nucleic acid sequences which hybridize under standard conditions with the nucleic acid sequence or fragments of the nucleic acid sequenc e having the function of the a promoter. It is advantageous to use short oligonucleotides of a length of 10 -50 bp, preferably 15-40 bp, for example of the conserved or other regions, which can be determined via comparisons with other related promoters in a manner known to the skilled worker for the hy bridization. Alternatively, it is also possible to use longer fragments of the nucleic acids according to the invention or the complete sequences for the hybridization. These standard conditions vary dependi ng on the nucleic acid used, viz. oligonucleotide, longer fragment or complete sequence, or depending on which type of nucleic acid, viz. DNA or RNA, is being used for the hy - bridization. Thus, for example, the melting temperatures for DNA: DNA hybrids ar e approx. 10°C lower than those of DNA: RNA hy brids of equal length.

Preferably standard conditions are understood to mean, depending on the nucleic acid, for example temperatures between 42°C and 58°C in an aqueous buffer solution with a concentration of between 0.1 and 5 x SSC (1 x SSC = 0.15 M NaCl, 15 mM sodium citrate, pH 7.2) or additionally in the presence of 50% formamide such as, for example, 42° C in 5 x SSC, 50% formamide. The hybridization conditions for DNA: DNA hybrids are advantageously 0.1 x SSC and temperatures of between approximately 20°C and 45°C, preferably between approx imately 30°C and 45°C. The hybridization conditions for DNA: RNA hybrids are advantageously 0.1 x SSC and temperatures of between approximately 30°C and 55°C, prefe rably between approximately 45°C and 55°C. These temperatures stated for the hybridization are melting temperature values which have been calculated by way of example for a nucleic acid with a length of approx. 100 nucleotides and a G + C content of 50% in the absence of formamide. The experimental conditions for DNA hybridization are described in specialist textbooks of genetics such as, for example, Sambrook et al.,"Molecular Cloning", Cold Spring Harbor Laboratory, 1989 and can be calculated using formul ae known to the skilled worker, for example as a function of the length of the nucleic acids, the type of the hybrids or the G + C content. The skilled worker can find more information on hybridization in the following text -books: Ausubel et al. (eds), 198 5, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Es - sential Molecular Biology: A Practical Approach, IRL Press at Oxford University Press, Oxford.

A functional equivalent is furthermore also understood to mean, in particular, natural or artificial mutations of the relevant nucleic acid sequences of the promoters as set forth in SEQ ID NO : 1 or SEQ ID NO : 8 and its homologs from other organisms, wherein muta - tions comprise substitutions, additions, deletions, inversions or insertions of one or more nucleotide residues.

The term "identity" or "homology" between two nucleic acid sequ ences is defined by the identity of the nucleic acid sequence/polypeptide sequence by in each case the entire sequence length, which is calculated by alignment with the aid of the program algorithm GAP (Wisconsin Package Version 10.0, University of Wiscons in, Genetics Computer Group (GCG), Madison, USA), setting the following parameters:

| | |
|---|---|
| Gap Weight: 8 | Length Weight: 4 |
| Average Match: 2,912 | Average Mismatch: -2, 003. |

Nucleic acid molecules which are advantageously for the process according to the invention can be isolated based on their homology to the nucleic acid molecules disclosed herein using the sequences or part thereof as hybridization probe and following standard hybridization techniques under stringent hybridization conditions. In this context, it is possible to use, for example, isolated nucleic acid molecules of at least 15, 20, 25, 30, 35, 40, 50, 60 or more nucleotides, preferably of at least 15, 20 or 25 nucleotides in length which hybridize under stringent conditions with the above -described nucleic acid molecules, in particular with those which encompass a nucleotide sequence of SEQ ID NO: 1 or SEQ ID NO: 8. Nucleic acid molecules with 30, 50, 100, 250 or more nucleotides may also be used.

The term homology when used herein is the same as t he term identity. Functional equivalents thus encompass naturally occurring vari ants of the sequences described herein, and also artificial, for example chemically synthesized, nucleic acid sequences.

The term "homology" means that the respective nuc leic acid mol ecules or encoded proteins are functionally and/or structurally equivalent. The nucleic acid molecules that are homologous to the nucleic acid molecules described above and that are derivatives of said nucleic acid molecules are, for example, variations of said nucleic acid mol ecules which represent modifications having the same biological function or substa ntially the same biological function. They may be naturally occurring variations, such as s equences from other fungi varieties or species, or mutations. These mutations may occur nat urally or may be obtained by mutagenesis techniques. The allelic variations may be naturally occurring allelic variants as well as synthetically produced or genetically engineered variants.

By "hybridizing" it is meant that such nucleic acid molecules hybridize under conve ntional hybridization conditions, preferably under stringent conditions such as described by, e.g., Sambrook (Molecular Cloning; A Laboratory Manual, 2nd Edition, Cold Spring Harbor Laboratory Press, Cold Spring Harbor, NY (1989)) or in Current Protocols in Molecular Biology, John Wiley & Sons, N. Y. (1989), 6.3.1 -6.3.6.

A preferred, nonlimiting example of stringent Southern blot hydridization conditions are hybridizations in 6 x sodium chloride/ sodium citrate (= SSC) at approximately 45°C, followed by one or more wash steps in 0.2 x SSC, 0.1% SDS at 50 to 65°C, for example at 50°C, 55°C or 60°C. The skilled worker knows that these hybridization conditions differ as a function of the type of the n ucleic acid and, for example when organic solvents are present, with regard to the temperature and concentration of the buffer. The temperature under "standard hybridization conditions" differs for example as a function of the type of the nucleic acid betw een 42°C and 58°C, preferably between 45°C and 50°C in an aqueous buffer with a concentration of 0.1 x 0.5 x, 1 x, 2x, 3x, 4x or 5 x SSC (pH 7.2). If organic solvent(s) is/are present in the abovementioned buffer, for example 50% formamide, the temperature under standard conditions is approximately 40°C, 42°C or 45°C. The hybridization conditions for DNA:DNA hybrids are preferably for example 0.1 x SSC and 20°C, 25°C, 30°C, 35°C, 40°C or 45°C, preferably between 30°C and 45°C. The hybridization conditions f or DNA:RNA hybrids are preferably for example 0.1 x SSC and 30°C, 35°C, 40°C, 45°C, 50°C or 55°C, preferably between 45°C and 55°C. The abovementioned hybridization temperatures are determined for example for a nucleic acid approximately 100 bp (= base pa irs) in length and a G + C content of 50% in the absence of formamide. The skilled worker knows to determine the hybridization conditions required with the aid of textbooks, for example the ones mentioned above, or from the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical A pproach", IRL Press at Oxford University Press, Oxford.

A further example of one such stringent hybridization condition is hybridization at 4XSSC at 65°C, followed by a was hing in 0.1XSSC at 65°C for one hour. Alternatively, an exemplary stringent hybridiz ation condition is in 50 % formamide, 4XSSC at 42°C. Further, the conditions during the wash step can be selected from the range of conditions delimited by low-stringency conditions (approximately 2X SSC at 50°C) and high - stringency conditions (approx imately 0.2X SSC at 50°C, preferably at 65°C) (20X SSC: 0.3M sodium citrate, 3M NaCl, pH 7.0). In addition, the temperature during the wash step can be raised from low -stringency conditions at room temperature, approx imately 22°C, to higher-stringency conditions at approximately 65°C. Both of the p arameters salt concentration and temperature can be varied simultaneously, or else one of the two parameters can be kept constant while only the other is varied. Denaturants, for example formamide or SDS, may also be em ployed during the hybridization. In the presence of 50% formamide, hybridization is preferably effected at 42°C. Relevant factors like i) length of treatment, ii) salt conditions, iii) detergent conditions, iv) competitor DNAs, v) temperature and vi) probe selection can combined case by case so that not all possibilities can be mentioned herein.

Some examples of conditions for DNA h ybridization (Southern blot assays) and wash step are shown hereinbelow:
(1) Hybridization conditions can be selected, for ex ample, from the following conditions:
   a) 4X SSC at 65°C,
   b) 6X SSC at 45°C,
   c) 6X SSC, 100 mg/ml denatured fragmented fish sperm DNA at 68°C,
   d) 6X SSC, 0.5% SDS, 100 mg/ml denatured salmon sperm DNA at 68°C,
   e) 6X SSC, 0.5% SDS, 100 mg/ml denatured f ragmented salmon sperm DNA, 50% formamide at 42°C,
   f) 50% formamide, 4X SSC at 42°C,
   g) 50% (vol/vol) formamide, 0.1% bovine serum albumin, 0.1% Ficoll, 0.1% polyvinylpyrrolidone, 50 mM sodium phosphate buffer pH 6.5, 750 mM NaCl, 75 mM sodium citrate at 42°C,
   h) 2X or 4X SSC at 50°C (low -stringency condition), or
   i) 30 to 40% formamide, 2X or 4X SSC at 42°C (low -stringency condition).
(2) Wash steps can be selected, for example, from the following conditions:
   a) 0.015 M NaCl/0.0015 M sodium citrate/0.1% SDS at 50°C.
   b) 0.1X SSC at 65°C.
   c) 0.1X SSC, 0.5 % SDS at 68°C.
   d) 0.1X SSC, 0.5% SDS, 50% formamide at 42°C.
   e) 0.2X SSC, 0.1% SDS at 42°C.
   f) 2X SSC at 65°C (low-stringency condition).
   g) 0,2XSSC, 0,1 %S DS at 60°C (medium -high stringency condit ions), or
   h) 0,1XSSC, 0,1 %S DS at 60°C (medium -high stringency conditions), or
   l) 0,2XSSC, 0,1 %S DS at 65°C (high stringency conditions), or
   h) 0,1XSSC, 0,1 %S DS at 65°C (high stringency conditions).

Further, some applications have to be performed at lo w stringency hybrid isation conditions, without any consequences for the specificity of the hybridisation. For exa mple, a Southern blot analysis of total DNA could be probed with a nucleic acid molecule of the present invention and washed at low stringency (55°C in 2xSSPE, 0,1% SDS). The hybridisation analysis could r eveal a simple pattern of only genes encoding polypeptides of the present invention, e.g. having herein -mentioned compound X increasing activity and/or having also the biological activity of pro tein X. A fu rther example of such low - stringent hybridization conditions is 4XSSC at 50°C or hybridization with 30 to 40% formamide at 42°C. Such molecules co mprise those which are fragments, analogues or derivatives of the polypeptide of the invention and differ, for example, by way of amino acid and/or nucleotide deletion(s), insertion(s), substitution (s), addition(s) and/or recombination (s) or any other modificat ion(s) known in the art either alone or in combination from the above -described amino acid sequences or their u nderlying nucleotide sequence(s). However, it is preferred to use high stringency h ybridisation conditions.

Hybridization should advantageously be carried out with fragments of at least 5, 10, 15, 20, 25, 30, 35 or 40 bp, advantageousl y at least 50, 60, 70 or 80 bp, preferably at least 90, 100 or 110 bp. Most preferably are fragments of at least 15, 20, 25 or 30 bp. Preferably are also hybridizations with at least 100 bp or 200, very especially preferably at least 400 bp in length. In a n especially preferred embodiment, the hybridization should be carried out with the entire nucleic acid sequence with conditions described above.

The introduction, into the organisms in question (transformation, transfection), fungi or a fungal cell, of a promoter of the invention and/or a gene construct of the invention and/or one expression cassette accor ding to the invention and/or at least one vector according to the invention can be e ffected in principle by all methods with which the skilled worker i s familiar.

The term "transformation" describes in the present context a process for introducing heterologous DNA into fungi or a fungal cell. The term transformed cell or fungi describes not only the product of the transformation process per se, but also all of the transgenic progeny of the transgenic organism generated by the transformation.

In the present case of fungi, the skilled worker will find suitable methods in the tex tbooks by Sambrook, J. et al. (1989)"Molecular cloning: A laboratory manual", Cold Spring Harbor Laboratory Press, by F.M. Ausubel et al. (1994) "Current protocols in molecular biology", John Wiley and Sons, by D.M. Glover et al., DNA Cloning Vol.1, (1995), IRL Press (ISBN 019 -963476-9), by Kaiser et al. (1994) Methods in Yeast G enetics, Cold Spring Habor Laboratory Press or Guthrie et al. "Guide to Yeast Genetics and Molecular Biology", Methods in Enzymology, 1994, Academic Press. In the case of the transformation of filamentous fungi, methods of choice are firstly the generation of protoplasts and transformation with the aid of PEG (Wiebe et al. (1997) Mycol. Res. 101 (7): 971-877; Proctor et al. (1997) Microbiol. 143, 2538 -2591) and secondly the transformation with the aid of Agrobacterium tumefaciens (de Groot et al. (1998) Nat. Biotech. 16, 839-842).

The transgenic fungi generated by transformation with one of the above -described embodiments of an expression cassette according to the invention or with a vector according to the invention are subject matter of the present invention.

In a further embodiment of the invention the method of the invention and the above described promoters, gene constructs, expression cassettes and vectors can be used for the expression or overexpression of at least one nucleic acid or one of the genes wh ich code for proteins involved in the metabolism.

In particular said genes code for proteins involved in fine chemical synthesis. Thus the invention provides a means to increase the yield of commercially important metabolites by genetic manipulation of the fungal organism itself. This facilitates the large scale production of fungal products which to date has not been possible.

The term "fine chemical" defines certain products and by -products of naturally-occurring metabolic processes in cells have utility in a wide array of industries, including the food, feed, cosmetics, and pharmaceutical industries. These molecules, collectively termed "fine chemicals", include organic acids, both proteinogenic and non -proteinogenic amino acids, nucleotides and nucleos ides, lipids and fatty acids, carotenoids, diols, carbohydrates, aromatic compounds, vitamins and cofactors and enzymes. Preferably fine chemicals include amino acids as for example Ala, Arg, Asn, Asp, Cys, Glu, Gln, Gly, His, Ile, Leu, Lys, Met, Phe, Pro , Ser, Thr, Trp, Tyr and Val or vitamins as for example vitamin A, D, E, K, B1, B2, B6, B12, C, pantothenic acid, biotin or folic acid; substances with vitamin-like character for example vitamin F, lipoic acid, ubiquinones, choline, myoinsositiol, vitamin U (S-methylmethionine), flavours for example vanillin, coumarin, isoeugenol, eugenol, (R) -carvone, (S)-carvone, menthol, jasmone or famesol; nutraceuticals for example phytosterols, flavonoids, anthocyanidins, isoflavons or isoprenoids; detergents; fatty acids such as saturated fatty acids, mono unsaturated fatty acids (singular MUFA, plural MUFAS), poly unsaturated fatty acids (singular PUFA, plural PUFAS), waxes or lipids containing said fatty acids; carbohydrates for example cellulose, starch, dextrin, pectin, xanthangum, carrageenan or alginate; sugars for example monosaccharides such as glucose, fructose, manose, sorbose, ribose, ribulose, xylose, xylulose or galactose, disaccharides such as lactose, sucrose, saccharose, maltose, isomaltose or cellobi ose, trisaccharides such as raffinose or maltotriose; carboxylic acids for example citric acid, α-ketoglutaric acid, ferulic acid, sinapic acid or lactic acid; carotinoids for example α-carotene, β - carotene, zeaxanthine, lutein, astaxanthine, lycopene, phyotoene or phytofluene, amino acids for example lysine, threonine, methionine, tryptophane, phenylalanine or tyrosine, cofactors for example heme or quinines, enzymes for example lipase s, esterases, cellulases, proteases, amylases, glucosidases etc. as well as other products (e.g. gibberellic acid, natural pigments, and flavorings) and other compounds [as described e.g. in Kuninaka, A. (1996) Nucleotides and related compounds, p. 561 -612, in Biotechnology vol. 6, Rehm et al., eds. VCH: Weinheim, in Industial Microbiology and Biotechnology, Demain et al., second edition, ASM Press Washington, D.C. 1999, in Ullmann's Encyclopedia of Industrial Chemistry, vol. A27, Vitamins, p. 443-613 (1996) VCH: Weinheim and Ong, A.S., Niki, E. & Packer, L. (1995) Nutrition, Lipids, Health, and Disease Proceedings of the UNESCO/Confederation of Scientific and Technological Associations in Malaysia, and the Society for Free Radical Research, Asia, held Sept. 1-3, 1994 at Penang, Malaysia, AOCS Press, (1995)), enzymes, and all other chemicals described in Gutcho (1983) Chemicals by Fermentation, Noyes Data Corporation, ISBN: 0818805086 and references contained therein]. In addition the transformed fungi of the present inventen may produce pharmaceutical active compounds such as antibiotics (e.g., beta -lactam antibiotics such as penicillin, cephalosporin, and their derivatives), anti -hypercholesterolemic agents (e.g., lovastatin and compactin), immunosuppressive s (e.g., cyclosporin), and antifungal drugs (e.g., pneumocandin and echinocandin).

The terms "increased", "rised", "extended", "enhanced", "improved" or "amplified" relate to a corresponding change of a property in a fungi. Preferrably, the overall activi ty in the volume is increased or enhanced in cases if the increase or enhancement is related to the increase or enhancement of an activity of a gene product, independent whether the amount of gene product or the specific activity of the gene product or bot h is increased or enhanced or whether the amount, stability or translation efficacy of the nucleic acid sequence or gene encoding for the gene product is increased or enhanced. The terms "reduction", "decrease" or "deletion" relate to a corresponding chang e of a property in fungi. Preferrably, the overall activity in the volume is reduced, decreased or deleted in cases if the reduction, decrease or deletion is related to the reduction, decrease or deletion of an activity of a gene product, independent wheth er the amount of gene product or the specific activity of the gene product or both is reduced, decreased or deleted or whether the amount, stability or translation efficacy of the nucleic acid sequence or gene encoding for the gene product is reduced, decr eased or deleted.

Accordingly, the term "increase" or "decrease" means that the specific activity of an enzyme as well as the amount of a compound or metabolite, e.g. of a polypeptide, a nucleic acid molelcule or of a fine chemical or an encoding mRNA or D NA, can be increased or decreased in a volume.

The terms "wild type", "control" or "reference" are exchangeable and can be a cell or a part of organisms such as an organelle or a tissue, or an organism, in a fungi, which was not modified or treated accor ding to the herein described process according to the invention. Accordingly, the cell or a part of organisms such as an organelle or a tissue, or an organism, in particular a fungi used as wild typ, control or reference corresponds to the cell, organism o r part thereof as much as possible and is in any other property but in the result of the process of the invention as identical to the subject matter of the invention as possible. Thus, the wild type, control or reference is treated identically or as identi cal as possible, saying that only conditions or properties might be different which do not influence the quality of the tested property.

Preferably, any comparison is carried out under analogous conditions. The term "analogous co nditions" means that all c onditions such as, for example, culture or growing conditions, assay conditions (such as buffer composition, temperature, substrates, pathogen strain, concentrations and the like) are kept identical between the experiments to be compared.

In a prefered em bodiment according to the invention, the nucleic acid sequences I) used in the process according to the invention is expressed and/or overexpressed after induction only. The increase, decrease or modulation according to this invention induced after adding the inducer.

The term "expression" refers to the transcription and/or translation of a codogenic gene segment or gene. As a rule, the resulting product is an mRNA or a protein. However, expression products can also include functional RNAs such as, for exam ple, antisense, nucleic acids, tRNAs, snRNAs, rRNAs, RNAi, siRNA, ribozymes etc.

In a prefered embodiment, the expression of nucleic acid sequences according to the method of the invention leads directly to an increase of the fine chemical, for example by expressing or overexpreesing genes coding for the fine chemical or coding for enzymes involved directly in the synthesis of the fine chemical.

In an other prefered embodiment, the expression of nucleic acid sequences according to the method of the inventio n leads firstly to a decrease in certain gene expression in the fungal cell. The decrease of the expression of certain genes in the cell leads subsequently to an increase of the content of the fine chemical.

This reduction, decrease or deletion (reduction, decrease, deletion, inactivation or down - regulation shall be used as synonyms throughout the specification) can be by all methods known to the skilled person, preferably by double -stranded RNA interference (dsRNAi), introduction of an antisense nucleic ac id, a ribozyme, an antisense nucleic acid co mbined with a ribozyme, a nucleic acid encoding a co -suppressor, a nucleic acid encoding a dominant negative protein, DNA - or protein-binding factors targeting said gene or -RNA or - proteins, RNA de gradation indu cing viral nucleic acids and expression systems, systems for indu cing a homolog recombination of said genes, mutations in said genes or a combination of the above.

A reduction in the activity or the function is preferably achieved by a reduced expression of a gene. Further preferred embodiements of the invention to reduce the biological activity or function of those genes can be achieved u sing the following methods:
■ introduction of a double -stranded RNA nucleic acid s equence (dsRNA) as described above or of an expression cassette, or more than one expre ssion cassette, ensuring the expression of the latter;
■ introduction of an antisense nucleic acid sequence or of an e xpression cassette ensuring the expression of the latter. Enco mpassed are those methods in which the antisense nucleic acid sequence is directed against a gene (i.e. genomic DNA sequences) or a gene transcript (i.e. RNA sequences) including the 5'and 3'non - translated regions. Also e ncompassed are α-anomeric nucleic acid s equences.
■ introduction of an antisense nucleic acid sequence in combin ation with a ribozyme or
■ introduction of sense nucleic acid sequences for inducing c osuppression or
■ introduction of a nucleic acid sequence encoding dom inant-negative protein or
■ introduction of DNA-, RNA- or protein-binding factors against genes, RNA's or proteins or
■ introduction of viral nucleic acid sequences and expression co nstructs which bring about the degradation of RNA, or
■ introduction of constructs for inducing homologous recombin ation on endogeno us genes, for example for generating knockout m utants.

Preferably the term antisense nucleic acid sequence as described above, particular for double-stranded RNA interference (dsRNAi) comprises a construct comprising at least a fragment of the target gene in sense (5'-3' direction of transcription), meaning the desired endogenic gene whose activity is to be reduced, a loop, which may contain a selection marker, and at least a fragment of said target gene in antisense (3' -5' direction of transcription).

Thus, the promoters according to the invention can be used to suppress gene expression of any genes. If appropriate, even a sequence segment of as little as approximately 90 bp in length suffices for this purpose.

The validation of a cellular target for drug screening purposes generally involves an experimental demonstration that inactivation of that gene product leaves the cell inviable or apathogen. Accordingly to the above mentioned methods, in a prefered embodiment the process of the present invention, the above described promoters, gene constructs, expression cassettes and vectors can be used for the expression or overexpression of at least one nucleic acid can be used to identify targets for fungucides.

The transformed fungi can be further used for the i dentification of fungicides.

### Examples:

The MET3 genes of Saccharomyces cerevisiae and Candida albicans are predicted to encode ATP sulphurylase enzymes (EC 2.7.7.4). Previous studies in these organisms have shown that the expression of these genes, whose product catalyses the first committed step in the fixation of inorganic sulphur (reviewed by Marzluf et al., 1997a), is strongly repressed by the presence of the sulphur containing amino acid methionine (Cherest et al., 1985, Care et al., 1999). In S. cerevisiae methionine exerts a stringent repression of MET3 gene expression (Mountain et al., 1991). This observation led to the use of the promoter for control of gene expression in the aforementioned yeasts for regulated expression of test genes (Dir ick et al., 1995; Mao et al., 2002; Care et al., 1999).

Nitrate reductase (EC 1.7.1.1) catalyses the reduction of nitrate to nitrite as part of the nitrate assimilatory pathway. In many fungi nitrate is only used in the absence of preferred nitrogen sou rces such as ammonium, glutamine and glutamate and the expression of nitrate reductase encoding genes has been shown to be repressed by the presence of such preferentially used N -sources, a phenomenon referred to as nitrogen - catabolite repression(reviewed by Marzluf 1997b; Magasanik and Kaiser, 2002). Under these conditions nitrate activates the expression of genes required for nitrate assimilation including nitrate reductase (Jargeat et al., 2003; Cove, 1979; Banks et al., 1991; Fu and Marzluf, 1987a; Fu and Marzluf, 1987b).

The promoter from the M. grisea homologue of these genes can be used for expression vector construction or functional analysis.

### Materials and Methods

The plasmid EGFP-1 was obtained from BD Biosciences. pCAMBIA0380 was purchased from CAMBIA technologies Canberra Australia. Magnaporthe grisea 70 -15 was purchased from Fungal Genetics Stock Centre, Kansas, USA. Real -time RT-PCR was carried out using a Roche Light -Cycler and Qiagen dye chemistry

### Construction of MET3p::GFP expressing Magnaporthe grisea strains

The enhanced green fluorescent protein -encoding gene EGFP was excised from plasmid pEGFP-1 by digestion with Xhol and Xbal and sub -cloned into pBluescript SK - to create plasmid pAJF-GFP. The MET3 promoter (appendix 1) was amplif ied from Magnaporthe grisea genomic DNA using the primers
Met3a ctc caa ccc gca ata gca cga cac (SEQ ID NO:2)
Met3b tcc ggg cgg tag aca tcc tca act (SEQ ID NO: 3)

The primers were predicted to amplify a 2332 bp amplicon. A circa 2.3kb band was obtained and cloned into the vector pGEM -Teasy (Promega). Partial sequencing of the plasmid obtained (pGEM -MET3p) confirmed that the product was indeed derived from the MET3 promoter region. A second PCR was used to introduce an Ncol site at the position of the first codon in the predicted product of the MET3 gene using the primers
MET3CC CCC CGA GCA CAA CGA CGA CCA CGC (SEQ ID NO: 4)
MET3NCO GGC CAT GGT TGG CGT GCG TGA GGG ATT G (SEQ ID NO: 5)

These primers are nested to the original pair and were predicted to giv e rise to a 1550 bp product. The primer contains two mismatches (underlined), which alter two base pairs in the final promoter sequence as shown below:
Original sequence ATCGCACAATCCCTCACGCACGCCAA TAATGGCC (SEQ ID NO: 6)
Final sequence ATCGCACAATCCCTCACG CACGCCAACCATGGCC SEQ ID NO: 7)

The altered bases are underlined and the first codon of predicted MET3 product is shown in bold. The product of the amplification was cloned into pGEM -T easy to give pGEM - MET3pNco. pGEM-MET3pNco was digested with EcoRI and Sp el and the insert excised and sub-cloned into pBluescript SK-to give pBlue -MET3. pBlue-MET3 was then digested with Clal and Ncol and sub cloned into the Clal Ncol digested pAJF -GFP to give rise to pMET3-GFP. The plasmid pMET3 -GFP was sequenced to confirm that it did indeed comprise the MET3 promoter cloned in frame with EGFP gene (so that the first codon of the EGFP gene is at the same position as that of the predicted native MET3 open reading frame). The MET3p -GFP insert was then excised using Apal and Spel and cloned into Apal-Spel digested pCAMB -HPT (a derivate of pCAMBIA0380 which carries the hygromycin resistance cassette from pCB1004 (Carrol et al., 1994) cloned as an Hpal fragment) to give rise to pCAMB -MET3-GFP. pCAMB-MET3-GFP was then transformed into Agrobacterium tumefaciens strain AGL1 and the T -DNA was transferred to M. grisea 70-15 conidiospores using Agrobacterium tumefaciens mediated transformation ATMT (Rho et al., 2001). Hygromycin resistant transformants were isolated and screened for GFP fluorescence using fluorescence microscopy.

### Construction of NOR1 p::GFP expressing M. grisea strains

The NOR1 promoter was amplified from M. grisea genomic DNA using the primers
NORP-C GGTGCCGCCGTTCGCCGTCTTAGTA (SEQ ID NO: 9)
NORP-D GTCGCCGCCCGCCTTCTT CACA (SEQ ID NO: 10)

The primers were predicted to amplify a 5182 bp amplicon. An approximately 5.2 kb band was obtained and cloned into the vector pGEM -T easy (Promega). Partial sequencing of the plasmid obtained (pGEM -NOR1 p) confirmed that the product was indeed derived form the NOR1 promoter region.

pGEM-NOR1p was digested with Clal and Ncol and sub -cloned into Clal-Ncol digested pAJF-GFP to give rise to pNOR1 -GFP. The plasmid pNOR1 -GFP was sequenced to confirm that it did indeed contain the NOR1 pr omoter cloned in frame with EGFP. The NOR1-GFP insert was then excised using Apal and BamHI and cloned into Apal -BamHI digested pCAMB -HPT to give rise to pCAMB -NOR1-GFP. pCAMB -NOR1-GFP was then transformed into Agrobacterium tumefaciens strain AGL1 and th e T-DNA (containing the NOR1 promoter-EGFP fusion) was transferred to M. grisea strain 70 -15 using ATMT. Hygromycin resistant transformants were isolated and screened for GFP fluorescence.

### Results

A 2.3 kb putative MET3 promoter region including the pr edicted start of translation site (based on conceptual translation) was amplified. The promoter was fused in frame with the EGFP gene by altering the sequence at the translation start site. The sequence of the promoter and the EGFP gene within this const ruct determined and the in frame fusion was verified by sequence analysis. Twelve M. grisea transformants expressing the EGFP gene under the control of the MET3 promoter were isolated and subjected to further analysis. Methionine and cysteine were found t o repress strongly the level of GFP fluorescence in these strains which express GFP driven by a MET3 promoter. These results indicate that attenuated EGFP transcript levels result from the repression of MET3 promoter activity as has previously been report ed in Candida albicans (Care et al., 1999) and S. cerevisiae (Cherest et al., 1985; Mountain et al., 1991). Real time RT -PCR analysis was used to confirm that the presence of methionine and cysteine leads to attenuated EGFP transcript abundance in M. gris ea MET3-EGFP expressing strains (see Figure 2). These data are consistent with the view that the reduced fluorescence in the presence of met and cys is caused by differences in transcript abundance, and more specifically by reduced MET3 promoter activity, and hence reduced transcription of the fused gene in the presence of these amino acids.

A 5.3 kb putative NOR1 promoter region including the predicted start of translation site (based on conceptual translation) was amplified. A 2333 bp sub -clone of this promoter region was fused in frame with the EGFP gene using a naturally occurring Ncol site already present at the site of the first codon of the native predicted nitrate reductase - encoding gene. The sequence of the promoter and EGFP within this construc t determined and the in frame fusion was verified by sequence analysis. Twelve M. grisea transformants expressing the EGFP gene under the control of the NOR1 promoter were isolated and subjected to further analysis. Nitrate were found to induce strongly t he level of GFP fluorescence in these strains when compared to strains incubated in the absence of nitrate or using an alternative nitrogen source (see Figure 3). These results indicate that increased EGFP transcript levels in the presence of nitrate resu It from the induction of NOR1 promoter activity and hence the up -regulated transcription of the fused gene in the presence of nitrate. These data are consistent with observations made in a number of other fungi where nitrate has been found to induce the e xpression of nitrate reductase - encoding genes (Banks et al., 1991; Fu and Marzluf, 1987a; Fu and Marzluf, 1987b )

## Claims

1. A method for expressing a nucleotide sequence in fungi, said method comprising transforming a fungi with a transformation gene construct comprising
I) at least one nucleotide sequence and
II) a promoter functionally linked to the nucleotide sequ ence I), comprising
a) a nucleotide sequence as set forth in SEQ ID NO:1 and/or
b) a nucleotide sequence as set forth in SEQ ID NO:8 and/or
c) a fragment of (a) or (b) that retains the promoter activity of SEQ ID NO:1 and/or SEQ ID NO:8 and/or
d) functional equivalent nucleic acid molecule of the nucleic acid sequence set forth in (a) to (c) which has at least 50% identity with the nucleic acid molecule of (a) to (c) and/or
e) nucleic acid molecule which encompasses a nucleic acid molecule which is obtained by amplifying nucleic acid molecules from a cDNA library or a genomic library using the primers in SEQ ID NO: 2 and 3 or SEQ ID NO: 4 and 5 or SEQ ID NO: 9 and 10 and/or
f) nucleic acid molecule which is obtainable by screening a suitable nucleic acid library under stringent hybridization conditions with a probe comprising one of the sequences of the nucleic acid molecule of (a) to (e) or with a fragment thereof having at least 10 -15 nt, preferably 16 -20 nt, 21-30 nt, 31-50 nt, 51-100 nt, 101-200 nt or 201-500 nt of the nucleic acid molecule **characterized in** (a) to (e)
and
wherein the promoter II) drives the expression of the nucleotide sequence I) in a fungi.

2. The method of claim 1 or 2, wherein the fungi is a filamentous fungi.

3. The method of cl aim 1 or 2, wherein the a filamentous fungi is an Acremonium, Aspergillus, Aureobasidium, Cryptococcus, Filibasidium, Fusarium, Humicola, Magnaporthe, Mucor, Myceliophthora, Neocallimastix, Neurospora, Paecilomyces, Penicillium, Schizophyllum, Talaroinyces , Thermoascus, Thielavia, Tolypocladium, or Trichoderma.

4. The method as claimed in any of claims 1 to 3, wherein the fungi is a Magnaporthe species.

5. The method as claimed in claim 4, wherein Magnaporthe species include Magnaporthe rhizophila, Magnaporthe salvinii, Magnaporthe poae and Magnaporthe grisea, preferably Magnaporthe grisea.

6. The method as claimed in any of claims 1 to 5, wherein the promoter as set forth in SEQ ID NO:1 and SEQ ID NO: 8 are from Magnaporthe grisea.

7. The method as claimed in any of claims 1 to 5, wherein the nucleotide sequence I) is an antisense nucleic acid sequence.

8. A promoter from Magnaporthe grisea comprising
a) a nucleotide sequence as set forth in SEQ ID NO:1 and/or
b) a nucleotide sequence as set forth in SEQ ID NO:8 and/or
c) a fragment of (a) or (b) that retains the promoter activity of SEQ ID NO:1 and/or SEQ ID NO:8 and/or
d) functional equivalent nucleic acid molecule of the nucleic acid sequence set forth in (a) to (c) which has at least 50% identity with the nucleic acid molecule of (a) to (c) and/or
e) nucleic acid molecule which encompasses a nucleic acid molecule which is obtained by amplifying nucleic acid molecules from a cDNA library or a genomic library using the primers in SEQ ID NO: 2 and 3 or SEQ ID NO: 4 and 5 or SEQ ID NO: 9 and 10 and/or
f) nucleic acid molecule which is obtainable by screening a suitable nucleic acid library under stringent hybridization conditions with a probe comprising one of the sequences of the nucleic acid molecule of (a) to (e) or with a fragment thereof having at least 10 -15 nt, preferably 16-20 nt, 21-30 nt, 31-50 nt, 51-100 nt, 101-200 nt or 201-500 nt of the nucleic acid molecule **characterized in** (a) to (e).

9. A gene construct comprising
I) at least one nucleotide sequ ence and
II) a promoter according to claim 8 functionally linked to the nucleotide sequence I),
wherein the promoter II) drives the expression of the nucleotide sequence I) in a fungi.

10. An expression cassette comprising
I) at least one nucleotide seque nce and
II) a promoter according to claim 8,
III) at least one functional element
wherein all three elements are functionally linked together.

11. An expression cassette according to claim 10 additionally comprising
IV) a terminator which facilitates trans cription termination, preferably in fungi, which is functionally linked to the nucleic acid sequence III).

12. A transformation vector comprising
I) at least one nucleotide sequence and
II) a promoter according to claim 8 functionally linked to the nucleot ide sequence I),
wherein the promoter II) drives the expression of the nucleotide sequence I) in a fungi.

13. A transformation vector comprising an expression cassette according to claim 10 or 11.

14. A method of expressing heterologous genes in fungi compris ing the steps of
a) recombinantly transforming fungal cells in which it is desired to express said heterologous gene with said heterologous gene, in operative association with the promoter of claim 8;
b) allowing the fungal material so transformed to express su ch heterologous gene; and
c) detecting such expression.

15. A transgenic fungi comprising a gene construct as claimed in claim 8 or an expression cassette as claimed in any of claims 10 or 11 or a vector as claimed in any of claims 12 or 13.

16. Use of the a promo ter as claimed in claim 8, a gene construct as claimed in claim 8, an expression ca ssette as claimed in any of claims 10 or 11 or a vector as claimed in any of claims 12 or 13 in a process for identifying targets for fungicides.

17. Use of the transgenic or ganism as claimed in claim 15 in a process for identifying targets for fungicides.

18. Use of the a promoter as claimed in claim 8, a gene construct as claimed in claim 8, an expression ca ssette as claimed in any of claims 10 or 11 or a vector as claimed in any of claims 12 or 13 in a process for the production of fine chemicals.

19. Use of the transgenic organism as claimed in claim 15 in a process for the production of fine chemicals.

20. Use of the a promoter as claimed in claim 8, a gene construct as claimed in claim 8, an expression ca ssette as claimed in any of claims 10 or 11 or a vector as claimed in any of claims 12 or 13 in a process for decreasing the expression of a certain endogenic gene by introducing a antisense nucleic acid sequence of said endogenic gene.


SEQUENCE LISTING

<110>  BASF AG

<120>  Method for expressing a nucleotide sequence in fungi

<130>  AE 720-04 EP

<160>  10

<170>  PatentIn version 3.2

<210>  1
<211>  1550
<212>  DNA
<213>  Magnaporthe grisea

<220>
<221>  promoter
<222>  (60)..(1550)

<400>  1
ggccatggtt ggcgtgcgtg agggattgtg cgatgggagt tcaaggtaat gtggtggatt      60

agaagttggt tcacgcggat gctaagagat taatgtgtgt tctttccgag tatcactttc     120

cacacgactt ggaaagtaaa gtaaaactat aaagatgttc acaggatgaa atggacagac     180

ggagcgtttt agttcaaaag aatctatttc taaacggcgg cactatgccg ggtaaaaaaa     240

aagctaacag cgggtcttat acaggctgga tcgtcgccgg cggcttctcg cggtatgtaa     300

tccatcgtat gggatgctta ttagtcgcga tgacgtgctc atcgcaactt tttgtgtaga     360

ccaactggca cttgccaaag gctaaccaac cacttggcga gtcgtgtata tcaggttcgt     420

tacccttggc gtgggatgga gagaaaccga agagaacccg ctgcgaagct agcccctcca     480

tcagtgaagg ctttgatagc tcccagctgc ttcatttgcc ccgccgcggt tctgttgctt     540

agcgggcggg aggataccct ccccccgcaa tgacgtgctc ccatttccac ttgtgcgtct     600

agacattcat tcagccctgg atctgtttcg cctttgaacc acaaggccct ctttatcgga 660

agagacttga ctgccgcacc cgggaattga ccagcaatac gctgttcatt ccgtggaaaa 720

gtagactttt tatgtggtaa aacttccagt tcccttatta cccgccgata gccgccggcc 780

cgtggttcgt cggtcggtcg ggtggtcggt gccggtattt gtctagattg attgcaccat 840

agcttggttt tattcatcgg agccttcaca aacgcggcaa ccaaacagag agaattgatc 900

cttggccatt gctaggcaaa ataatatcac ggattcaatt tgtagcatta tgtctcctat 960

ctcacccttc caaaatcggc aacacagaac aacaaaatat tcccccaaaa aggccagcta 1020

aacttcaatt tgttaatccc tcaagggctc caaaacagca aacaagattg gacagataaa 1080

aaaaacagct gcatagggaa aatgtaaatc tttcttgcca catacttggt aaccggagca 1140

aacgatcctt caatcaagcg atatcaaggt tggcagagga gcccaaatct ccatcttttc 1200

catgttattg gtgctgtcgc tccagatcat gacccatagg cgggtagcct ggtcctaggc 1260

gcccgccgct cgtggctatg ctgatactgc gagacgcggc cgaggctgat atgatttctg 1320

tcaaaaatgt ggttgcgcca tctagcaatt catgtcagtc tttggctatg attacctgcg 1380

cagctccctc tttccttgaa ctgagacaaa taaaaacaat tggaactcac cccaacttgt 1440

tgtcgagcct gaccaccctg gcatgtcgcc agagctactc gtcgatgacg gcgactcagg 1500

agcatccgcg aagactgttg cgatgtgcgt ggtcgtcgtt gtgctcgggg 1550

<210> 2
<211> 24
<212> DNA
<213> Artificial

<220>
<223> Primer

<220>
<221>  Primer
<222>  (1)..(24)

<400>  2
ctccaacccg caatagcacg acac                                              24

<210>  3
<211>  24
<212>  DNA
<213>  Artificial

<220>
<223>  Primer

<400>  3
tccgggcggt agacatcctc aact                                              24

<210>  4
<211>  26
<212>  DNA
<213>  Artificial

<220>
<223>  Primer

<220>
<221>  Primer
<222>  (1)..(26)

<400>  4
ccccccgagc acaacgacga ccacgc                                            26

<210>  5
<211>  28
<212>  DNA
<213>  Artificial

<220>
<223>  Primer

<220>
<221>  Primer
<222>  (1)..(28)

<400>  5
ggccatggtt ggcgtgcgtg agggattg                                          28

<210>  6
<211>  34
<212>  DNA
<213>  Artificial

<220>
<223>  Primer

<220>
<221>  Primer
<222>  (1)..(34)

<400>  6
atcgcacaat ccctcacgca cgccaataat ggcc                                   34

<210>  7
<211>  34
<212>  DNA
<213>  Artificial

<220>
<223>  Primer

<220>
<221>  primer
<222>  (1)..(34)

<400>  7
atcgcacaat ccctcacgca cgccaaccat ggcc                                    34

<210>  8
<211>  2334
<212>  DNA
<213>  Magnaporthe grisea

<220>
<221>  promoter
<222>  (60)..(2334)

<400>  8
atcgattttc tcggaagctg tgacttgcag gtgatttatg gttgccagcc gcttggtccc      60

tacctcaacc ccgattaccc cttggtgacg tcttgtgtca aacgatacct tcccaaacct     120

ccctgctcaa gtcacccctt cctaggtacc ttaggtaagt gcctgggtaa ggtaggtatg     180

tacctaggta tcttgaggaa gatgaggcaa ggtaagccag tattaggcat ttgcaggtgt     240

gcacaacata ttctgtttaa gcaagggcat caggacctat gagcaatatc aaaagtcgcg     300

gcaggagact aggcgacaac acgggcctgt atttaaccac ttgctttgat ggctccaaag     360

aggcatatgt ccaataatta ttgattcata gccggaaaat gcaccccgta ctcagaagga     420

ctcatttgtt cgaagattta ttgtacagtg tccgctgaca gtgaaacaga gcatatattc     480

tcaattttca acgcctacgt aacttgtttg atgcgacatt gcgtcgatcc gggagtctat     540

tgccgaagcc gcgatgagat ccgggaatct ccttatttag cccaagcacc ggttgcctac     600

taaagtgcca ttcgtgcttg catacttcgt actgtacgac ctttcggtga gaggcaataa     660

ttattggaag ctgccatctc caatccaacg gtggggaata cccgacaggt ccggaaccga     720

ttaggtaagg caagatgaag taagtggtgg caccaccaca gtaggtaggt acgtgtggta     780

ggtaccttgc ccaactgact ggctccacta attttgttgg tgcctttgcg atcaaaaggc 840

gaaaaagaaa caaataaaat gagaagaaag atctctcgag caacaattga tctcaatggt 900

catatgatta cctcttttac tgatggcgga tatgtcgatc aatcataacc ccccggattt 960

tcgtttttag aacaaaaagc cattcaatcg cggagaaact cctgaaccga tcgcgacagc 1020

gatcgtgctc ggattctggg gatggggaaa cttatttccc tccacttcag ctgatgaatg 1080

actgaccggt ctctaaatcc tcttatctct aacccgcggc atgggtgtgg tcggactgcc 1140

gctgaagaag tttcaatcca ccacaaatta tagtaaccaa ctttcgtacc aagcagatca 1200

tgacgaggtc atccaagact gccacgaata cccgggtacc ttaatcagta cgcaatcgag 1260

cccaaccagg aatggttcta ggtagctcaa gtggtgtggg cagcagacgg gggatcagcc 1320

agcaccgcct atctttggtc gtccaatccc ctgggagcgg gtcgtcttat caacaacatg 1380

tctgattcca gtttcgctac ctcaaagata acggccgatc caagtggcca gcatcgtaat 1440

taagttgcag ccaagcaaga ttgtttactg ataagaccgc tccaatgcat agggctggct 1500

ggctgcctgc ctgcctgcat cacaagacca agcatttggc tagcacgcac gtccgcggaa 1560

ttgtcctata aaaatctact atctttgccc cccggagatg gataattcgc ccaagactcc 1620

cacgcaccat ctcccgtctt tcatcttccg acagaagaca tgcgctgcta tgtagtagag 1680

tgacattgac cggtacgcgc cgaaattcta gggctagttt tttaccgtat tggtcgacag 1740

cctgagtgag gtcaactcgc ccccatgccc ggcacttgcg atggagagcc tatggcttgg 1800

gctcatgtcg tagttgtcag gtcaagtcgc tgtctaaggt agttagcagt ttggtgcacg 1860

ttcactggct gcagatggcc acacccagag aaatatctcc aggtttccac catacggtat 1920

agacatccgc caagcctttg tctccttatg tccttctccc acacagcaaa tccctcgcaa 1980

cccataaaac acctcactta acatttatta cccaatcggc tctccttcag gtatatccaa 2040

gtgtgcttaa aacccgagta accacaccac tttcaagggt atgccagcat aaatatcatt 2100

gactaccgag accaatgaca aatgaataga tacctccaag ctagctttgc ctagtgagca 2160

atacctgcgt cttcttgtaa tctcttgtca tatactgaag acgtcatact cactcctatc 2220

ttaactgctt acacttctca ctccaccttg ttatagacct cgcaacactt ccgctacata 2280

catctttttc catagctact tcaaatcatc gctggtttcc tacacatatt ccaa 2334

<210> 9
<211> 25
<212> DNA
<213> Artificial

<220>
<223> Primer

<220>
<221> primer
<222> (1)..(25)

<400> 9
ggtgccgccg ttcgccgtct tagta 25

<210> 10
<211> 22
<212> DNA
<213> Artificial

<220>
<223> Primer

<220>
<221> Primer
<222> (1)..(22)

<400> 10

gtcgccgccc gccttcttca ca 22